# EUROPÄISCHE PATENTSCHRIFT

(11) **EP 0 572 790 B1**
(45) Veröffentlichungstag und Bekanntmachung des Hinweises auf die Patenterteilung: **14.02.1996**
(21) Anmeldenummer: 93106520.5
(22) Anmeldetag: 22.04.1993
(51) Int. Cl.: A61B 17/60

(54) **Osteosynthetisches Befestigungselement**
Osteosynthesis anchoring element
Elément de fixation d'ostéosynthèse

(30) Priorität: 04.06.1992 CH 799/92
(43) Veröffentlichungstag der Anmeldung: 08.12.1993
(73) Patentinhaber: Synthes AG, Chur, CH-7002 Chur (CH)
(72) Erfinder: Schläpfer, Johannes Fridolin, Dr., CH-8750 Glarus (CH); Flückiger, Markus, CH-4437 Waldenburg (CH)
(74) Vertreter: Lusuardi, Werther Giovanni, Dr.

(56) Entgegenhaltungen:
- EP-A- 0 348 272
- EP-A- 0 441 729
- WO-A-91/16020
- GB-A- 2 157 179

## Beschreibung

Die Erfindung bezieht sich auf ein osteosynthetisches Befestigungselement, insbesondere eine Pedikelschraube oder einen Wirbelsäulenhaken gemäss der Gattung des Patentanspruchs 1.

Ein derartiges Befestigungselement ist aus der GB-A-2 157 179 bekannt. Bei dem bekannten Befestigungselement ist ein Fixationselement mit konkaver Aushöhlung am unteren Ende beschrieben, das auf einen vom Längsträger durch bohrten Zylinder wirkt.

Aus der DE-U-89.15.443 ist bereits ein Befestigungselement dieser Gattung, insbesondere für die Wirbelsäulenchirurgie bekannt. Es besteht im wesentlichen aus einem am Knochen verankerbaren unteren Teil in Form eines Schraubenschaftes oder einer Klinge und einem daran anschliessenden, oberen Körper zur Befestigung an einer Stange, wobei in dem Körper ein nach oben mündender Kanal ausgebildet ist, der zwei seitliche Schenkel begrenzt, zwischen denen die Stange aufgenommen werden kann. Die Fixation der Stange innerhalb des Kanals erfolgt durch einen von oben zwischen die Schenkel einschraubbaren Gewindestopfen, dessen unteres, zur Anlage an die Stange bestimmtes Ende mit Verhakungsmitteln in Form einer oder mehrerer Spitzen versehen ist.
Die beim Eindrehen des Gewindestopfens zwischen die beiden Schenkel des Körpers an der darin liegenden Stange zur Anlage kommenden Spitzen graben sich punktuell in die Oberfläche der Stange ein und bewirken eine gewisse Fixierung der Stange relativ zum Körper. Nachteilig bei dieser Konstruktion ist der bloss punktuelle Kontakt zwischen Stange und Gewindestopfen, der sich leicht lockern kann, wenn Kräfte zwischen Stange und Befestigungselement zur Wirkung kommen, was im Wirbelsäulenbereich die Regel ist. Die langen Hebelarme mit denen an der Stange angreifende Kräfte auf die bloss punktuellen Verhakungen wirken, führen auch bei geringsten Kraftbeträgen zu einer raschen und irreversiblen Lockerung der Fixation.

Hier will die Erfindung für Abhilfe sorgen. Der Erfindung liegt die Aufgabe zugrunde, ein osteosynthetisches Befestigungselement der beschriebenen Gattung zu schaffen, welches bei auftretenden Kräften die Verklemmung aufrecht erhalten kann oder gar einen selbstklemmenden Effekt erzeugen kann, so dass eine Lockerung der aneinander befestigten Elemente verhindert wird.

Die Erfindung löst die gestellte Aufgabe mit einem Befestigungselement, welches die Merkmale des Anspruchs 1 aufweist.

Bei der Erfindung ist das untere Ende des Fixationselementes mit einem, vorzugsweise beweglich in einer Aushöhlung angeordneten Kugelteil versehen, der teilweise daraus hervorragt. Gegenüber den aus dem Stand der Technik bekannten Fixationselementen, insbesondere deren unteres Ende, hat diese Konstruktion den Vorteil, dass sie sich bei allfällig auftretenden Relativbewegungen zwischen den befestigten Elementen selbsttätig adaptieren kann ohne eine Lockerung zu bewirken. Innerhalb relativ breiter Grenzen bewirkt eine Relativbewegung zwischen Längsträger und Befestigungselement keine Lockerung. Dank der abrollbaren Kugelgeometrie kann eine bleibende, konstante Klemmung aufrecht erhalten werden.

Vorzugsweise ist der Kugelteil bloss als Kugelsegment oder Kugelschicht ausgebildet, damit statt einer bloss punktuellen eine lineare Auflage und Verklemmung erzielt werden kann.

Ist der Kugelteil beweglich ausgebildet, so versucht sich dieser bei einer allfällig auftretenden Translation zwischen Kugelteil und Längsträger aufgrund der Reibungskraft zu drehen, was zu einer Verkantung und damit sogar zu einer Erhöhung der Verklemmung führt.

Eine weitere Verbesserung kann erzielt werden, indem das Kugelteil mit einer konkaven, senkrecht zum Radius des Kugelteils liegenden, kreiszylindrischen Ausfräsung zur formschlüssigen Anlage an den Längsträger versehen wird. Damit kann ein dauerhafter Flächenkontakt erzielt werden.

Bei einer weiteren Ausführungsform der Erfindung ist das Kugelteil mit einer konkaven, senkrecht zum Radius des Kugelteils liegenden, sphärischen Ausfräsung zur formschlüssigen Anlage an eine auf den Längsträger aufschiebbare sphärische Manschette versehen. Wenn sich die sphärische Manschette - aufgrund einer am Längsträger angreifenden Kraft - in irgend eine Richtung drehen will, so versucht sich der Kugelteil mit der sphärischen Ausfräsung aufgrund der Reibung in die entgegengesetzte Richtung zu drehen, was wiederum zu einer Erhöhung der Klemmwirkung führt.

Eine weitere Verbesserung der Wirkung des erfindungsgemässen Befestigungselementes kann dadurch erzielt werden, dass auch die untere Auflage des Längsträgers auf dem Boden des Durchgangskanals adaptierbar gestaltet wird. Zu diesem Zweck ist der Boden des Sackloches mit einer konkaven, kreiszylinderförmigen Oberfläche ausgebildet, deren Zylinderachse senkrecht zur Längsachse und durch das Zentrum der Kugel verläuft. Zwischen dem Boden und dem Längsträger kann nun ein Kippelement eingelegt werden, welches einerseits eine zur Oberfläche des Sackloches korrespondierende, konvexe Oberfläche und anderseits eine zur Oberfläche des Längsträgers korrespondierende, konkave, kreiszylinderförmige Oberfläche aufweist, deren Zylinderachse senkrecht dazu verläuft. Mittels dieses zusätzlichen Kippelementes, welches um die gleiche Achse wie die Kugel rotieren kann, ist innerhalb eines erheblichen Winkelbereiches eine vollständige Adaptation an allfällig auftretende Winkeländerungen zwischen Längsträger und Befestigungselement möglich, ohne dass dabei die Verklemmung zwischen den einzelnen Elementen gelockert wird.
Bei dieser Ausführung wird der Boden des Sackloches vorzugsweise strukturiert ausgebildet, z.B. in Form einer Verzahnung oder durch eine keilförmige Geometrie. Auch die konvexe Oberfläche des Kippelementes kann analog strukturiert werden. Bevorzugte Kombinationen der beiden aneinander zur Anlage kommenden Oberflächen sind die folgenden:

| Kippelement | Sacklochboden |
|---|---|
| - verzahnt / hartes Material | - glatt / weiches Material |
| - glatt / weiches Material | - verzahnt / hartes Material |
| - verzahnt | - verzahnt |
| - keilförmige Geometrie der Längsseiten | - keilförmige Geometrie der Längsseiten |

Vorzugsweise ist der Durchgangskanal nach oben offen ausgebildet, derart dass der obere Abschnitt eine U-förmige Aufnahme mit zwei seitlichen Schenkeln für den Längsträger bildet. Bei dieser Ausführungsform werden die beiden Schenkel vorteilhafterweise mittels einer Stabilisierungshülse gesichert, da die beiden Schenkel beim Eindrehen des Fixationselementes die Neigung haben sich zu öffnen. Für spezielle Anwendung kann der Durchgangskanal aber auch oben geschlossen bleiben, so dass keine Stabilisierungshülse nötig ist.

Ausführungsbeispiele der Erfindung, welche zugleich das Funktionsprinzip erläutern, sind in der Zeichnung dargestellt und werden im folgenden näher beschrieben.
Fig. 1 stellt eine perspektivische Darstellung des Befestigungselementes mit einer Kugel als wesentlichem Fixationselement dar;
Fig. 2 stellt eine perspektivische Darstellung einer erfindungsgemäßen Ausführungsform mit einem Kugelteil als wesentlichem Fixationselement dar;
Fig. 3 stellt eine perspektivische Darstellung einer erfindungsgemäßen Ausführungsform mit einem zusätzlichen Kippelement dar;
Fig. 4 stellt einen teilweisen Längsschnitt durch das geschlossene Fixationselement nach Fig. 3 dar;
Fig. 5 stellt eine perspektivische Darstellung des Kippelementes nach Fig. 3 dar;
Fig. 6 stellt einen Querschnitt durch eine weitere Ausführungsform der Erfindung mit einer zusätzlichen Stabilisierungshülse dar; und
Fig. 7 stellt einen teilweisen Längsschnitt durch eine weitere Ausführungsform mit zusätzlicher Manschette für den Längsträger dar.

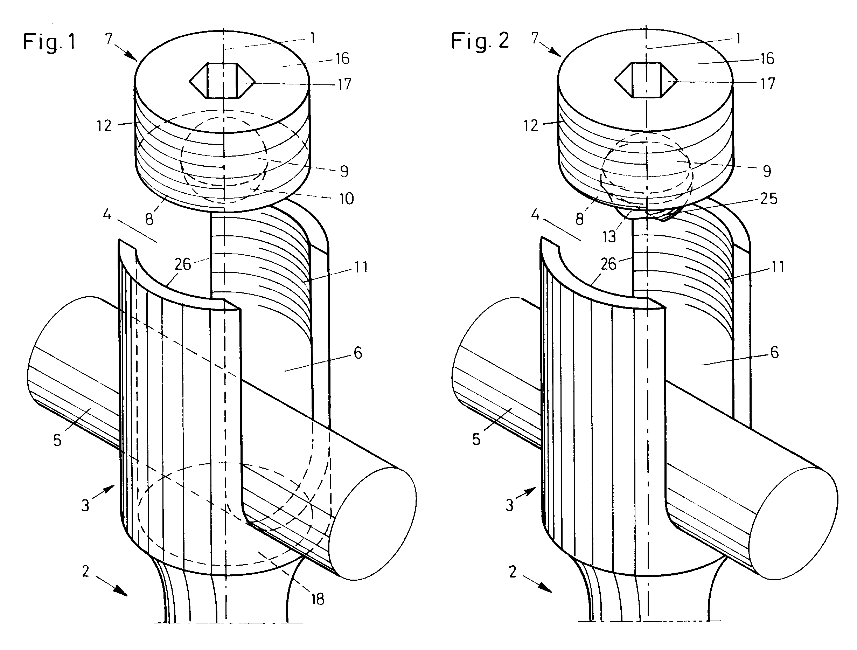

Fig. 1 zeigt eine perspektivische Darstellung des Befestigungselementes, welches im wesentlichen aus einem am Knochen verankerbaren unteren Abschnitt 2 und einem in Richtung der Längsachse 1 des Befestigungselementes daran anschliessenden, oberen Abschnitt 3 besteht. Der untere Abschnitt 2 kann als Schraubenschaft oder als abgebogene Klinge ausgebildet sein; im ersten Fall ergibt sich eine sogenannte Pedikelschraube im zweiten Fall ein Wirbelsäulenhaken.

Der obere Abschnitt 3 weist einen nach oben offenen, quer zur Längsachse 1 verlaufenden Durchgangskanal 4 auf, wodurch eine U-förmige Aufnahme mit zwei gegenüberliegenden Schenkeln 26 und einem Boden 18 gebildet wird, in welche der Längsträger 5 bequem eingeführt werden kann. Der obere Abschnitt 3 weist ferner ein in Richtung der Längsachse 1 verlaufendes, kreiszylindrisches, ebenfalls nach oben offenes Sackloch 6 auf. Im Inneren des Sackloches 6, d.h. an der Innenseite der beiden Schenkel 26, ist ein Innengewinde 11 vorgesehen. Zur Verschliessung des Sackloches 6 und Verklemmung des Längsträger 5 zwischen den beiden Schenkeln 26 ist ein kreiszylindrisches Fixationselement 7 vorgesehen, welches ein mit dem Innengewinde 11 korrespondierendes Aussengewinde 12 trägt.
Das Fixationselement 7 weist an seinem unteren, zur Anlage am Längsträger 5 bestimmten Ende 8 eine kugelsegmentförmige Aushöhlung 9 auf, in welcher ein Kugelteil 10 drehbar eingepasst ist, die teilweise aus der Aushöhlung 9 herausragt.
An seinem oberen Ende 16 weist das Fixationselement 7 eine in Richtung der Längsachse 1 verlaufende, polygonale Bohrung 17 auf, in welche ein geeignetes, formschlüssiges Instrument, z.B. ein Sechskantschlüssel einführbar ist, um das Fixationselement 7 in das Innengewinde 11 zwischen den beiden Schenkeln 26 eindrehen und gegen den Längsträger 5 festklemmen zu können.

In Fig. 2 ist eine Ausführungsform des erfindungsgemässen Befestigungselementes dargestellt, bei der erfindungsgemäß statt einer vollen Kugel bloss ein Kugelteil 25, nämlich eine Kugelschicht vorgesehen ist. Das Kugelteil 25 ist entweder flach ausgebildet oder mit einer konkaven, senkrecht zu seinem Radius liegenden, kreiszylindrischen Ausfräsung 13 zur formschlüssigen Anlage an den Längsträger 5 versehen. Die konkave Oberfläche 13 ist naturgemäss an die Oberflächengeometrie des zu verwendenden Längsträgers 5 anzupassen, d.h. sie muss die gleiche Krümmung aufweisen um einen optimalen Effekt zu erzielen, der darin besteht, dass eine dauerhafte Kontaktfläche zwischen der Oberfläche 13 des Kugelteils 25 und dem Längsträger 5 hergestellt wird.

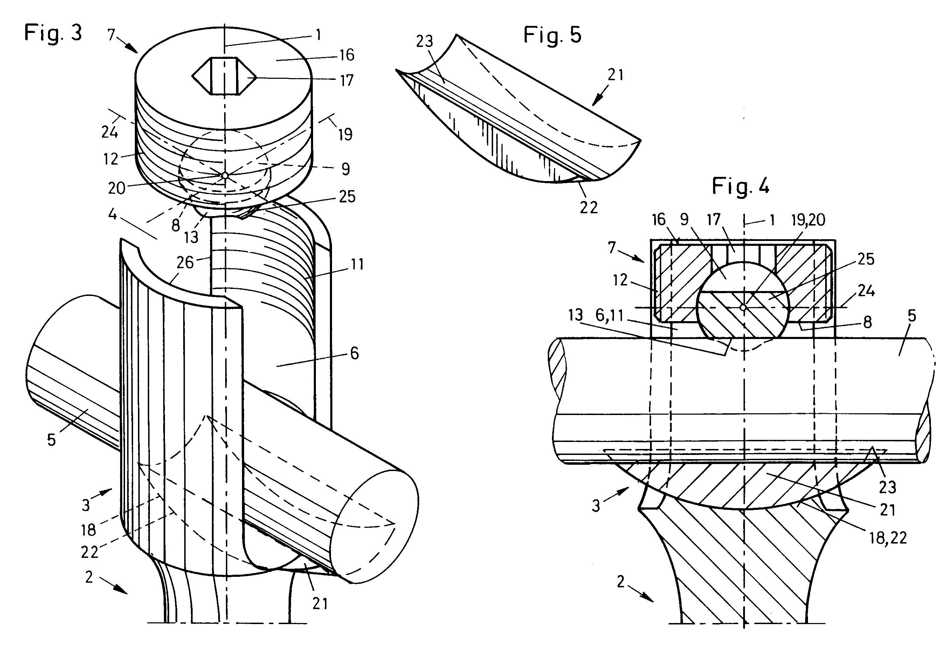

Die Fig. 3 - 5 zeigen eine weitere Variante des erfindungsgemässen Befestigungselementes, bei welcher der Boden 18 des Sackloches 6 eine konkave, kreiszylinderförmige Oberfläche aufweist, deren Zylinderachse 19 senkrecht zur Längsachse 1 und durch das Zentrum 20 des Kugelteils 25 hindurch verläuft. Ein zusätzliches, zwischen dem Boden 18 und dem Längsträger 5 einlegbares Kippelement 21 erlaubt eine koaxiale Rotation gleichzeitig mit dem Kugelteil 25. Das Kippelement 21, welches im Detail in Fig. 4 dargestellt ist, weist eine zur Oberfläche des Sackloches 18 korrespondierende, konvexe Oberfläche 22 und anderseits eine zur Oberfläche des Längsträgers 5 korrespondierende, konkave, kreiszylinderförmige Oberfläche 23 auf, deren Zylinderachse 24 senkrecht zur Zylinderachse 19 verläuft. Das Rotationszentrum des Kippelementes 21 ist identisch mit dem Zentrum 20 der kugeligen Aushöhlung 9 im Fixationselement 7, wenn das Fixationselement 7 angezogen ist.

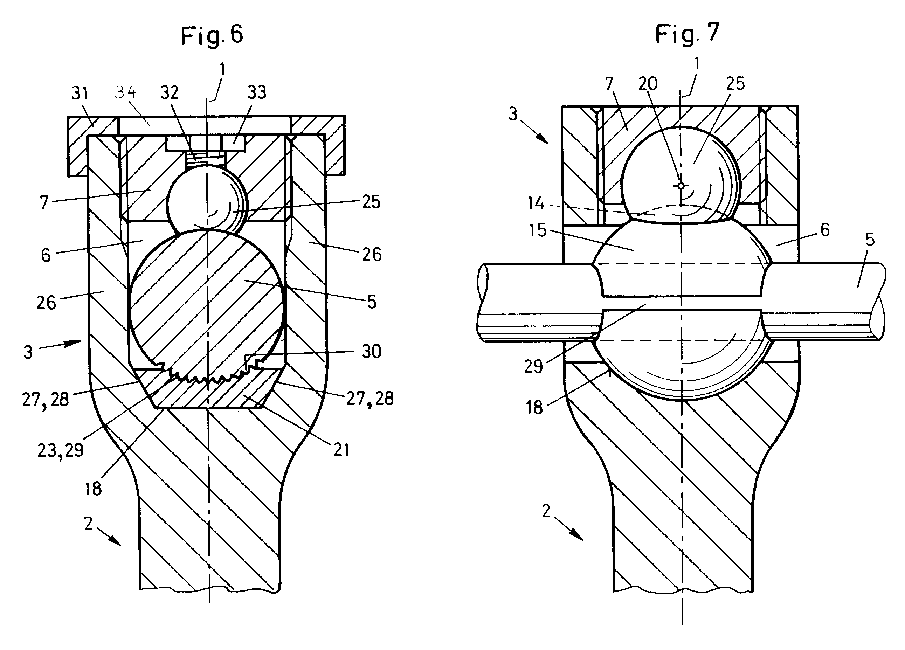

Fig. 6 zeigt eine weitere Variante des erfindungsgemässen Befestigungselementes, bei welcher der Boden des Sackloches 18 des oberen Abschnitts 3 in Richtung des Durchgangskanals 4 mit keilförmigen Abstufungen 27 versehen ist; dementsprechend sind die äusseren Längsseiten 28 des Kippelementes 21 ebenfalls keilförmig ausgebildet.
Die konkave Oberfläche 23 des Kippelementes 21 ist mit einer Längszähnung 29 versehen, welche mit einer entsprechenden Längszähnung 30 des Längsträgers 5 korrespondiert, so dass nach erfolgtem Anziehen des Fixationselementes 7 der Längsträger 5 gegen Verdrehung gesichert ist.
Schliesslich weist der obere Abschnitt 3 eine über die beiden Schenkel 26 stülpbare Stabilisierungshülse 31 mit einer zentralen Bohrung 34 auf, welche die beiden Schenkel 26 gegen eine Aufweitung, wie sie sich beim Eindrehen und Anziehen des Fixationselementes 7 ergeben kann, sichert.
Statt einer polygonalen Bohrung 17 (Fig. 1 - 3) ist bei dieser Ausführungsform eine Bohrung mit Innengewinde 32 und einem Querschlitz 33 zur Aufnahme eines schraubenzieherähnlichen Instrumentes, vorgesehen. Eine solche Gestaltung erleichtert das Einbringen, die Manipulation und das simultane Anziehen des Fixationselementes 7.

Fig. 7 zeigt schliesslich eine weitere Variante des erfindungsgemässen Befestigungselementes, bei welcher das Kugelteil 25, hier in Form eines Kugelsegmentes, mit einer konkaven, senkrecht zum Radius des Kugelteils 25 liegenden, sphärischen Ausfräsung 14 zur formschlüssigen Anlage an eine auf den Längsträger 5 aufschiebbare sphärische Manschette 15 versehen ist. Die Manschette 15 weist eine zentrale Bohrung mit einem Schlitz 29 auf um ein federndes Aufstecken auf dem Längsträger 5 zu gestatten. Auch bei dieser Ausführungsform müssen die sphärische Oberfläche der Manschette 25 und die konkave Ausfräsung 14 aufeinander abgestimmt sein, d.h. die gleiche Krümmung aufweisen.

## Patentansprüche

1. Osteosynthetisches Befestigungselement, insbesondere Pedikelschraube oder Wirbelsäulenhaken, mit einer Längsachse (1), mit einem am Knochen verankerbaren unteren Abschnitt (2) und einem in Richtung der Längsachse (1) daran anschliessenden, oberen Abschnitt (3), welcher von einem quer zur Längsachse (1) verlaufenden Durchgangskanal (4) zur Aufnahme eines Längsträgers (5) vollständig durchquert ist, und welcher ein in Richtung der Längsachse (1) verlaufendes, kreiszylindrisches, nach oben offenes Sackloch (6) zur kraftschlüssigen Aufnahme eines damit korrespondierenden, kreiszylindrischen Fixationselementes (7) für den Längsträger (5) aufweist, dadurch gekennzeichnet, dass das Fixationselement (7) an seinem unteren, dem Längsträger (5) gegenüberliegenden Ende (8) eine kugelsegmentförmige Aushöhlung (9) aufweist, in welcher ein teilweise daraus herausragendes Kugelteil (25) eingepasst ist, dessen nicht-sphärischer Teil zur direkten Anlage am Längsträger (5) bestimmt ist.

2. Befestigungselement nach Anspruch 1, dadurch gekennzeichnet, dass das sackloch (6) mit einem Innengewinde (12) und das Fixationselement (7) mit einem damit korrespondierenden Aussengewinde (12) versehen ist.

3. Befestigungselement nach Anspruch 1 oder 2, dadurch gekennzeichnet, dass das Kugelteil (25) drehbar in der Aushöhlung (9) gelagert ist.

4. Befestigungselement nach einem der Ansprüche 1 - 3, dadurch gekennzeichnet, dass das Kugelteil (25) als Kugelsegment oder Kugelschicht ausgebildet ist.

5. Befestigungselement nach Anspruch 4, dadurch gekennzeichnet, dass das Kugelteil (25) mit einer konkaven, senkrecht zum Radius des Kugelteils (25) liegenden, kreiszylindrischen Ausfräsung (13) zur formschlüssigen Anlage an den Längsträger (5) versehen ist.

6. Befestigungselement nach Anspruch 4, dadurch gekennzeichnet, dass das Kugelteil (25) mit einer konkaven, senkrecht zum Radius des Kugelteils (25) liegenden, sphärischen Ausfräsung (14) zur formschlüssigen Anlage an eine auf den Längsträger (5) aufschiebbare sphärische Manschette (15) versehen ist.

7. Befestigungselement nach einem der Ansprüche 1 bis 6, dadurch gekennzeichnet, dass das Fixationselement (7) an seinem oberen Ende (16) eine in Richtung der Längsachse (1) verlaufende, polygonale Bohrung (17) zur formschlüssigen Aufnahme eines Instrumentes aufweist.

8. Befestigungselement nach einem der Ansprüche 1 bis 6, dadurch gekennzeichnet, dass das Fixationselement (7) an seinem oberen Ende (16) einen quer zur Längsachse (1) verlaufenden Schlitz (33) mit einer zentralen Bohrung mit Innengewinde (32) zur formschlüssigen Aufnahme eines Instrumentes aufweist.

9. Befestigungselement nach einem der Ansprüche 1 bis 8, dadurch gekennzeichnet, dass
A) der Boden (18) des Sackloches (6) eine konkave, kreiszylinderförmige Oberfläche aufweist, deren Zylinderachse (19) senkrecht zur Längsachse (1) und durch das Zentrum (20) der Kugel (10) oder des Kugelteils (25) verläuft, und
B) ein zusätzliches, zwischen dem Boden (18) und dem Längsträger (5) einlegbares Kippelement (21) vorgesehen ist, welches einerseits eine zur Oberfläche des Sackloches (18) korrespondierende, konvexe Oberfläche (22) und anderseits eine zur Oberfläche des Längsträgers (5) korrespondierende, konkave, kreiszylinderförmige Oberfläche (23) aufweist, deren Zylinderachse (24) senkrecht zur Zylinderachse (19) verläuft.

10. Befestigungselement nach einem der Ansprüche 1 bis 9, dadurch gekennzeichnet, dass der Boden (18) des Sackloches (6) sowie die konvexe Oberfläche (22) des Kippelementes (21) strukturiert, vorzugsweise mit einer Verzahnung, ausgebildet sind.

11. Befestigungselement nach einem der Ansprüche 1 bis 10, dadurch gekennzeichnet, dass die konkave Oberfläche (23) des Kippelementes (21) sowie die Oberfläche des Längsträgers (5) strukturiert, vorzugsweise mit einer Verzahnung (29,30), ausgebildet sind.

12. Befestigungselement nach einem der Ansprüche 1 bis 11, dadurch gekennzeichnet, dass der Durchgangskanal (4) nach oben offen ausgebildet ist, derart, dass der obere Abschnitt (3) eine U-förmige Aufnahme für den Längsträger (5) bildet.

13. Befestigungselement nach einem der Ansprüche 1 bis 12, dadurch gekennzeichnet, dass zusätzlich eine, vorzugsweise eine zentrale Bohrung (34) aufweisende Stabilisierungshülse (31) vorgesehen ist, welche formschlüssig auf die beiden Schenkel (27) stülpbar ist.

14. Befestigungselement nach einem der Ansprüche 1 bis 13, dadurch gekennzeichnet, dass das Kugelteil (25) mindestens gleich gross wie die korrespondierende Aushöhlung (9) ist.

15. Befestigungselement nach einem der Ansprüche 1 bis 14, dadurch gekennzeichnet, dass die Aushöhlung (9) eine gegenüber ihrem Grosskreis geringere Austrittsöffnung aufweist.

## Claims

1. Osteosynthetic fixation element, in particular a pedicle screw or a spinal column hook, with a longitudinal axis (1), a lower portion (2) that can be anchored into bone, and an upper portion (3) that adjoins the lower along the longitudinal axis (1), said upper portion (3) being fully breached by a passageway channel (4) running transverse to the longitudinal axis (1) to accommodate a longitudinal support (5), said upper portion (3) having a circular-cylindrical pocket hole (6) which runs parallel to the longitudinal axis (1) allowing frictional accommodation of a matching circular-cylindrical fixation part (7) for the longitudinal support (5),
characterized in that
the fixation part (7) is provided on its bottom side (8), which is meant to adjoin the longitudinal support (5), with a cavity (9) in the shape of a spherical segment, in which a spherical part (25) is fitted, partially protruding from the cavity (9), and whose non-spherical part is meant to take its direct bearing on the longitudinal support (5).

2. Osteosynthetic fixation element according to claim 1, characterized in that the pocket hole (6) is provided with an interior threading (11) and the fixation part (7) is provided with a matching exterior threading (12).

3. Osteosynthetic fixation element according to claim 1 or 2, characterized in that the spherical part (25) is rotatably held in the cavity (9).

4. Osteosynthetic fixation element according to one of the claims 1 - 3, characterized in that the spherical part (25) is in the form of a spherical segment or spherical layer.

5. Osteosynthetic fixation element according to claim 4, characterized in that the spherical part (25) is equipped with a concave circular-cylindrical countersinking (13), perpendicular to the radius of the spherical part (25), for an interlocking fit onto the longitudinal support (5).

6. Osteosynthetic fixation element according to claim 4, characterized in that the spherical part (25) is equipped with a concave spherical countersinking (14), perpendicular to the radius of the spherical part (25), for an interlocking fit onto a slip-on spherical collar (15) on the longitudinal support (5).

7. Osteosynthetic fixation element according to one of the claims 1 - 6, characterized in that the fixation part (7) has a polygonal borehole (17) on its top side (16), running parallel to the longitudinal axis (1), for interlockingly receiving a fitting tool.

8. Osteosynthetic fixation element according to one of the claims 1 - 6, characterized in that the fixation part (7) on its top side (16) has a slot (33) running transverse to the longitudinal axis (1), with a central borehole with interior threading (32), for interlockingly receiving a fitting tool.

9. Osteosynthetic fixation element according to one of the claims 1 - 8, characterized in that
A) the floor (18) of the pocket hole (6) has a concave, circular-cylindrical surface, whose cylindrical axis (19) is perpendicular to the longitudinal axis (1), and runs through the center (20) of the sphere (10) or the spherical part (25); and
B) an additional tipping part (21) is provided between the floor (18) and the longitudinal support (5), whereby on one of its sides the tipping part (21) has a convex surface (22) that matches the surface of the pocket hole (18), while the other side has a concave, circular-cylindrical surface (23) that fits the surface of the longitudinal support (5), the cylindrical axis (24) of this other side running perpendicular to the cylindrical axis (19).

10. Osteosynthetic fixation element according to one of the claims 1 - 9, characterized in that the floor (18) of the pocket hole (6) and the convex surface (22) of the tipping part (21) are provided with a structured surface, preferably in form of a toothing.

11. Osteosynthetic fixation element according to one of the claims 1 - 10, characterized in that the concave surface (23) of the tipping part (21) and the surface of the longitudinal support (5) are provided with a structured surface, preferably in form of a toothing (29, 30).

12. Osteosynthetic fixation element according to one of the claims 1 - 11, characterized in that the passageway channel (4) is formed so as to be open at the top in such a way that the upper portion (3) forms a U-shaped accommodation for the longitudinal support (5).

13. Osteosynthetic fixation element according to one of the claims 1 - 12, characterized in that additionally a stabilizer socket (31) is provided, preferably one which has a central borehole (34), which is mountable on the two flanks (27) in a form-fitting fashion.

14. Osteosynthetic fixation element according to one of the claims 1 - 13, characterized in that the spherical part (25) is at least as large as the corresponding cavity (9).

15. Osteosynthetic fixation element according to one of the claims 1 - 14, characterized in that the cavity (9) has an opening circle which is inferior to its great circle.

## Revendications

1. Elément de fixation pour ostéosynthèse, en particulier vis à pédicule ou crochet pour colonne vertébrale, comportant un axe longitudinal (1), une partie inférieure (2) pouvant être ancrée sur l'os et une partie supérieure (3) qui s'y raccorde dans la direction de l'axe longitudinal (1), et qui est traversée complètement par un canal de passage (4) s'étendant transversalement par rapport à l'axe longitudinal (1), pour la réception d'un support longitudinal (5), et qui présente un trou aveugle (6) ouvert vers le haut, cylindrique circulaire, s'étendant dans la direction de l'axe longitudinal (1), pour loger sous l'action d'une force un élément de fixation (7) correspondant, cylindrique circulaire, pour le support longitudinal (5), caractérisé en ce que l'élément de fixation (7) présente à son extrémité inférieure (8), opposée au support longitudinal (5), un évidement en forme de segment de sphère (9), dans lequel est adaptée une partie de sphère (25) qui en déborde partiellement, dont la partie non sphérique est destinée à une pose directe sur le support longitudinal (5).

2. Elément de fixation selon la revendication 1, caractérisé en ce que le trou aveugle (6) est pourvu d'un filet intérieur (12) et l'élément de fixation (7) d'un filet extérieur (12) correspondant.

3. Elément de fixation selon la revendication 1 ou 2, caractérisé en ce que la partie de sphère (25) est montée de manière à pouvoir tourner dans l'évidement (9).

4. Elément de fixation selon l'une quelconque des revendications 1-3, caractérisé en ce que la partie de sphère (25) est configurée comme segment de sphère ou segment sphérique tronqué.

5. Elément de fixation selon la revendication 4, caractérisé en ce que la partie de sphère (25) est pourvue d'un évidement concave fraisé (13), cylindrique circulaire, situé perpendiculairement au rayon de la partie de sphère (25), pour la pose en correspondance de forme sur le support longitudinal (5).

6. Elément de fixation selon la revendication 4, caractérisé en ce que la partie de sphère (25) est pourvue d'un évidement sphérique concave (14) situé perpendiculairement au rayon de la partie de sphère (25), pour la pose en correspondance de forme sur une manchette sphérique (15) qui peut être glissée sur le support longitudinal (5).

7. Elément de fixation selon l'une quelconque des revendications 1 à 6, caractérisé en ce que l'élément de fixation (7) présente à son extrémité supérieure (16) un alésage polygonal (17) s'étendant dans la direction de l'axe longitudinal (1), pour la réception en correspondance de forme d'un instrument.

8. Elément de fixation selon l'une quelconque des revendications 1 à 6, caractérisé en ce que l'élément de fixation (7) présente à son extrémité supérieure (16) une fente (33) s'étendant transversalement par rapport à l'axe longitudinal (1) et comportant un alésage central à filet intérieur (32), pour la réception en correspondance de forme d'un instrument.

9. Elément de fixation selon l'une quelconque des revendications 1 à 8, caractérisé en ce que
A) le fond (18) du trou aveugle (6) présente une surface cylindrique circulaire concave dont l'axe de cylindre (19) s'étend perpendiculairement à l'axe longitudinal (1) et passe par le centre (20) de la sphère (10) ou de la partie de sphère (25), et
B) il est prévu un élément supplémentaire d'inclinaison (21) pouvant être inséré entre le fond (18) et le support longitudinal (5), qui présente d'une part une surface convexe (22) correspondant à la surface du trou aveugle (18) et d'autre part une surface concave cylindrique circulaire (23), correspondant à la surface du support longitudinal (5), et dont l'axe de cylindre (24) s'étend perpendiculairement à l'axe de cylindre (19).

10. Elément de fixation selon l'une quelconque des revendications 1 à 9, caractérisé en ce que le fond (18) du trou aveugle (6) ainsi que la surface convexe (22) de l'élément d'inclinaison (21) sont de configuration structurée, de préférence avec une dentelure.

11. Elément de fixation selon l'une quelconque des revendications 1 à 10, caractérisé en ce que la surface concave (23) de l'élément d'inclinaison (21) ainsi que la surface du support longitudinal (5) sont de configuration structurée, de préférence avec une dentelure (29, 30).

12. Elément de fixation selon l'une quelconque des revendications 1 à 11, caractérisé en ce que le canal de passage (4) est configuré ouvert vers le haut, de telle sorte que la partie supérieure (3) forme un logement en forme de U pour le support longitudinal (5).

13. Elément de fixation selon l'une quelconque des revendications 1 à 12, caractérisé en ce qu'il est en plus prévu un manchon de stabilisation (31), présentant de préférence un alésage central (34), et qui peut être enfiché en correspondance de forme sur les deux ailes (27).

14. Elément de fixation selon l'une quelconque des revendications 1 à 13, caractérisé en ce que la partie de sphère (25) est au moins aussi grande que l'évidement correspondant (9).

15. Elément de fixation selon l'une quelconque des revendications 1 à 14, caractérisé en ce que l'évidement (9) présente une ouverture de sortie plus petite que son grand cercle.
